# EUROPÄISCHE PATENTANMELDUNG

(11) **EP 4 567 392 A1**
(43) Veröffentlichungstag der Anmeldung: **11.06.2025**
(21) Anmeldenummer: 24217158.5
(22) Anmeldetag: 03.12.2024
(51) Int. Cl.: G01L 5/16, G01L 3/14

(54) **SENSORANORDNUNG**

(30) Priorität: 04.12.2023 DE 102023133864
(71) Anmelder: Resense GmbH, 63911 Klingenberg (DE)
(72) Erfinder: Matich, Sebastian, 97980 Bad Mergentheim (DE)
(74) Vertreter: Zimmermann & Partner Patentanwälte mbB

(57) **Zusammenfassung**

Sensoranordnung (1) zur Messung einer Kraft und/oder eines Drehmoments, umfassend eine Mehrzahl von Sensorabschnitten (11) mit jeweils einem Sensor (13), wobei die Sensorabschnitte (11) um eine erste Achse (3) angeordnet ist; und eine Mehrzahl von Stegen (21), welche jeweils zwei benachbarte der Sensorabschnitte (11) miteinander verbinden, wobei eine Verbindung (33) zwischen einem Steg (21) und einem ersten Sensorabschnitt von benachbarten Sensorabschnitten in einem ersten axialen Bereich (23) bereitgestellt ist, und wobei sich der Steg (21) in einem Zwischenraum (17) zwischen den benachbarten Sensorabschnitten in einen von dem ersten axialen Bereich (23) verschiedenen zweiten axialen Bereich (25) erstreckt.

## Beschreibung

### Gebiet der Erfindung

Die Offenbarung betrifft eine Sensoranordnung zur Messung einer Kraft oder eines Drehmoments, und ein Verfahren zum Herstellen einer Sensoranordnung.

### Stand der Technik

Aus dem Stand der Technik sind Anordnungen von Sensoren bekannt, welche Kräfte oder Drehmomente messen können. Insbesondere für Anwendungen in der Medizintechnik, beispielsweise in Telemanipulatoren für minimalinvasive Chirurgie, oder in der Robotik, beispielsweise in industriellen Greifsystemen, können solche Anordnungen von Sensoren zur Messung oder Regelung von Kräften oder Drehmomenten genutzt werden. Beispielsweise kann eine Sensoranordnung zur Bereitstellung eines haptischen Feedbacks eines Greif- oder Manipulationsarms oder eines daran vorgesehenen Endeffektors dienen. Für zahlreiche Anwendungen sind möglichst kleine oder kostengünstige Kraft- und Drehmomentsensoren vorteilhaft.

Allerdings weisen bekannte Sensoranordnungen Restriktionen auf, insbesondere hinsichtlich einer Miniaturisierung der Sensoranordnungen. Alternativ dazu oder zusätzlich können bekannte Sensoranordnungen, insbesondere bei fortschreitender Verkleinerung der Sensoranordnungen, Restriktionen in Bezug auf eine Messgenauigkeit oder eine Stabilität der Sensoranordnung aufweisen oder einen hohen Aufwand in der Herstellung erfordern.

### Offenbarung der Erfindung

Aufgabe der Offenbarung ist es, eine Sensoranordnung zur Messung einer Kraft oder eines Drehmoments anzugeben, welche gegenüber dem Stand der Technik verbessert ist. Insbesondere sollte eine Sensoranordnung angegeben werden, welche besonders klein, stabil oder kostengünstig gebaut werden kann oder eine hohe Messgenauigkeit aufweist. Weiterhin soll ein Verfahren zum Herstellen einer Sensoranordnung angegeben werden.

Die Aufgabe wird mit einer Sensoranordnung zur Messung einer Kraft oder eines Drehmoments gemäß Anspruch 1 sowie mit einem Verfahren nach dem nebengeordneten Anspruch gelöst.

Gemäß einem Aspekt wird eine Sensoranordnung zur Messung einer Kraft oder eines Drehmoments angegeben, insbesondere zur Messung einer Kraft und eines Drehmoments. Die Sensoranordnung umfasst eine Mehrzahl von Sensorabschnitten mit jeweils einem Sensor, wobei die Sensorabschnitte um eine erste Achse angeordnet ist. Die Sensoranordnung umfasst eine Mehrzahl von Stegen, welche jeweils zwei benachbarte der Sensorabschnitte miteinander verbinden, wobei eine Verbindung zwischen einem Steg und einem ersten Sensorabschnitt von benachbarten Sensorabschnitten in einem ersten axialen Bereich bereitgestellt ist, und wobei sich der Steg in einem Zwischenraum zwischen den benachbarten Sensorabschnitten in einen von dem ersten axialen Bereich verschiedenen zweiten axialen Bereich erstreckt.

Gemäß einem weiteren Aspekt wird ein Verfahren zum Herstellen einer Sensoranordnung gemäß hierin beschriebenen Ausführungsformen angegeben. Das Verfahren umfasst ein Bereitstellen der Mehrzahl von Sensorabschnitten, welche über die Stege verbunden sind, wobei die Sensorabschnitte in einer Ebene angeordnet sind. Das Verfahren umfasst ein Aufrollen der Sensorabschnitte zur Anordnung der Sensorabschnitte um die erste Achse.

Gemäß typischen Ausführungsformen umfasst die Sensoranordnung eine Mehrzahl von Sensorabschnitten mit jeweils einem Sensor. Die Sensorabschnitte umfassen typischerweise jeweils einen Messkörper zur Aufnahme oder Übertragung von Kräften oder Drehmomenten. Typischerweise sind die Sensoren der Sensorabschnitte in oder auf den jeweiligen Messkörpern derart angeordnet, dass Messungen der Sensoren zur Bestimmung der auf die Sensoranordnung wirkenden Kräfte oder Drehmomente geeignet sind. Typischerweise sind die Sensoren flach oder planar ausgebildet und insbesondere flach auf dem Messkörper angeordnet.

Bei typischen Ausführungsformen sind die Sensoren der Sensorabschnitte Messelemente oder insbesondere Dehnmesselemente. Die Dehnmesselemente sind typischerweise jeweils zur Bestimmung einer Dehnung oder einer mechanischen Verspannung eines jeweiligen Sensorabschnitts eingerichtet, insbesondere eines Messkörpers des Sensorabschnitts. Das Dehnmesselement kann beispielsweise einen Folien-Dehnmessstreifen (DMS) oder einen Halbleiter-DMS umfassen. Ein Halbleiter-DMS kann insbesondere ein Silizium-DMS (Si-DMS) sein. Si-DMS können beispielsweise eine besonders kleine Baugröße aufweisen. Der Sensor eines Sensorabschnitts kann mindestens ein Dehnmesselement aufweisen, insbesondere genau ein Dehnmesselement oder genau zwei Dehnmesselemente. Die Sensoren sind typischerweise fest mit den jeweiligen Sensorabschnitten, insbesondere den jeweiligen Messkörpern, verbunden, beispielsweise verklebt, verlötet oder mittels Glaslot gefügt.

Typischerweise sind die Sensorabschnitte zumindest im Wesentlichen gleichartig ausgebildet, insbesondere mechanisch zumindest im Wesentlichen gleichartig ausgebildet. Unter "zumindest im Wesentlichen mechanisch gleichartig" ist insbesondere zu verstehen, dass die mechanischen Eigenschaften und die Form der Sensorabschnitte zumindest im Wesentlichen gleichartig sind. Die Sensorabschnitte können aber beispielsweise Unterschiede in einer elektrischen Verschaltung der Sensoren aufweisen, beispielsweise in einer Anzahl von Leiterbahnen, welche durch den Sensorabschnitt verlaufen. Die Mehrzahl von Sensorabschnitten können hierin auch als Mehrzahl von Elementarzellen bezeichnet werden. Die unter Verwendung der Sensoranordnung zu bestimmenden Kräfte oder Drehmomente können auf Basis einer Mehrzahl von Messungen der Sensoren der jeweiligen Sensorabschnitte bzw. Elementarzellen bestimmt werden.

Bei typischen Ausführungsformen sind die Sensorabschnitte um eine erste Achse angeordnet. Die Begriffe "axial", "radial" und "in Umfangsrichtung" beziehen sich hierin typischerweise auf die erste Achse. Typischerweise sind mindestens drei Sensorabschnitte um die erste Achse angeordnet, insbesondere mindestens vier oder mindestens 5, oder maximal 24, insbesondere maximal 20 oder maximal 16. Bei typischen Ausführungsformen sind die Sensorabschnitte rings um die erste Achse angeordnet. Beispielsweise können 3 Sensorabschnitte rings um die erste Achse angeordnet sein, insbesondere als Tripod. In weiteren Beispielen können 6, 8 oder 12 Sensorabschnitte rings um die erste Achse angeordnet sein. Beispielsweise kann eine Sensoranordnung mit 6 Sensorabschnitten als hexagonale Struktur angeordnet sein, insbesondere als Hexapodstruktur. In weiteren Ausführungsformen können die Sensorabschnitte in einer Schnecken- oder Spiralanordnung oder in einer Wabenstruktur um eine erste Achse angeordnet sein. Insbesondere eine Sensoranordnung mit Wabenstruktur kann eine Vielzahl von Sensorabschnitten umfassen, insbesondere auch mehr als 20 Sensorabschnitte.

Bei typischen Ausführungsformen umfasst die Sensoranordnung eine Mehrzahl von Stegen. Typischerweise verbinden die Stege jeweils zwei benachbarte Sensorabschnitte der Mehrzahl von Sensorabschnitten miteinander. Typischerweise sind die Stege jeweils in einem Zwischenraum zwischen zwei benachbarten Sensorabschnitten angeordnet. Bei Ausführungsformen verbinden die Stege die Sensorabschnitte mechanisch in Reihe miteinander. Insbesondere sind typischerweise jeweils zwei in der Reihe benachbarte Sensorabschnitte über einen Steg miteinander verbunden. Bei typischen Ausführungsformen umfassen die in Reihe verbundenen Sensorabschnitte einen Anfangssensorabschnitt und einen Endsensorabschnitt, welche nicht direkt durch einen Steg miteinander verbunden sind. Bei Ausführungsformen sind die über die Stege verbundenen Sensorabschnitte um die erste Achse aufgerollt angeordnet.

Gemäß typischen Ausführungsformen weisen die Stege eine geringere Dicke auf als die Sensorabschnitte. Typischerweise weisen die Stege jeweils maximal die Hälfte der Dicke eines Sensorabschnitts auf, beispielsweise maximal ein Drittel oder maximal ein Fünftel der Dicke eines Sensorabschnitts. Typischerweise bezieht sich die Dicke auf eine Dicke in einer radialen Richtung.

Bei typischen Ausführungsformen umfasst die Sensoranordnung eine flexible Leiterplatte. Die flexible Leiterplatte umfasst typischerweise Stegbereiche, welche die Stege der Sensoranordnung bilden. Bei Ausführungsformen umfasst die flexible Leiterplatte Sensorabschnittsbereiche, wobei die Sensorabschnitte der Sensoranordnung jeweils einen der Sensorabschnittsbereiche der flexiblen Leiterplatte umfassen. Typischerweise sind die Sensorabschnittsbereiche der flexiblen Leiterplatte jeweils als Teil eines Sensorabschnitts ausgebildet. Typischerweise sind die Sensorabschnittsbereiche jeweils fest mit einem Messkörper eines Sensorabschnitts verbunden. Beispielsweise kann ein Sensorabschnittsbereich der flexiblen Leiterplatte auf den Messkörper aufgeklebt sein oder an einem Sensor, der fest mit dem Messkörper verbunden ist, angelötet sein. Typischerweise weist der Messkörper eine größere Dicke auf als die flexible Leiterpatte, insbesondere in radialer Richtung. Beispielsweise kann der Messkörper mindestens doppelt so dick sein wie die flexible Leiterplatte, insbesondere mindestens dreimal oder mindestens fünfmal so dick. Die flexible Leiterplatte ist bei typischen Ausführungsformen ein Flexleiter, insbesondere ein Flexleiter auf Folienbasis. Die flexible Leiterplatte kann eine oder mehrere Lagen von Folien oder Leiterbahnen aufweisen. Beispielsweise kann die flexible Leiterplatte zwei Leiterlagen aufweisen. Die Dicke der flexiblen Leiterplatte kann kleiner als 0,5 mm sein, insbesondere kleiner als 0,3 mm oder kleiner als 0,2 mm, beispielsweise ungefähr 0,1 mm.

Typischerweise weisen die Sensorabschnittsbereiche der flexiblen Leiterplatte jeweils eine Verdrahtungszone zur Verdrahtung des Sensors des jeweiligen Sensorabschnitts auf. Eine Verdrahtungszone eines Sensorabschnittsbereichs kann beispielsweise Leiterbahnen oder Kontaktpads zur elektrischen Verbindung mit dem Sensor des Sensorabschnitts aufweisen. Typischerweise weisen die Stege jeweils Leiterbahnen zur elektrischen Verbindung der Mehrzahl von Sensorabschnitten auf. Bei typischen Ausführungsformen sind die Sensorabschnittsbereiche und die Stege durch genau eine flexible Leiterplatte ausgebildet, insbesondere so, dass die genau eine flexible Leiterplatte alle Sensorabschnitte der Sensoranordnung miteinander verschaltet.

Bei typischen Ausführungsformen ist eine Verbindung zwischen einem Steg und einem ersten Sensorabschnitt von zwei benachbarten Sensorabschnitten in einem ersten axialen Bereich, der sich in Richtung der ersten Achse erstreckt, bereitgestellt. Die Verbindung zwischen dem Steg und dem ersten Sensorabschnitt kann beispielsweise durch den Übergang zwischen dem auf dem Messkörper des ersten Sensorabschnitts angeordneten Sensorabschnittsbereich einer flexiblen Leiterplatte und dem in einem Zwischenraum zwischen den benachbarten Sensorabschnitten angeordneten Steg gebildet werden. Typischerweise erstreckt sich der Steg in dem Zwischenraum zwischen den benachbarten Sensorabschnitten in einen von dem ersten axialen Bereich verschiedenen zweiten axialen Bereich. Typischerweise ist eine weitere Verbindung zwischen dem Steg und einem zweiten Sensorabschnitt der benachbarten Sensorabschnitte in dem ersten axialen Bereich bereitgestellt. Bei weiteren Ausführungsformen kann die weitere Verbindung in einem von dem ersten axialen Bereich verschiedenen, weiteren axialen Bereich vorgesehen sein. Die weitere Verbindung zwischen dem Steg und dem zweiten Sensorabschnitt ist typischerweise analog zu der Verbindung zwischen dem Steg und dem ersten Sensorabschnitt ausgebildet. Bei typischen Ausführungsformen kann durch die Erstreckung eines Stegs in den zweiten axialen Bereich vorteilhafterweise der Weg des Kraftflusses zwischen benachbarten Sensorabschnitten verlängert werden, was insbesondere zu einer erhöhten Flexibilität des Stegs führen kann.

Gemäß typischen Ausführungsformen ist eine axiale Erstreckung des zweiten axialen Bereichs größer als eine axiale Erstreckung des ersten axialen Bereichs. Bei Ausführungsformen ist eine axiale Erstreckung des zweiten axialen Bereichs größer als eine Erstreckung des Stegs in einer Richtung senkrecht zu der ersten Achse. Bei typischen Ausführungsformen erstrecken sich die Stege jeweils in axialer Richtung über mindestens ein Viertel, insbesondere mindestens ein Drittel, einer axialen Länge eines Sensorabschnittes. Bei typischen Ausführungsformen erstrecken sich die Stege in dem zweiten axialen Bereich jeweils in axialer Richtung über mindestens ein Viertel einer axialen Länge eines Sensorabschnittes. Bei Ausführungsformen sind die Verbindung und die weitere Verbindung zwischen einem Steg und den benachbarten Sensorabschnitten an einem dem Sensor des Sensorabschnitts näheren axialen Ende eines Sensorabschnittsbereichs einer flexiblen Leiterplatte angeordnet. Bei weiteren Ausführungsformen sind die Verbindung und die weitere Verbindung an einem von dem Sensor des Sensorabschnitts weiter entfernten axialen Ende des Sensorabschnittsbereichs der flexiblen Leiterplatte angeordnet.

Bei Ausführungsformen weisen die Stege jeweils einen sich axial erstreckenden ersten Längsabschnitt und einen sich axial erstreckenden zweiten Längsabschnitt auf. Typischerweise sind der erste Längsabschnitt und der zweite Längsabschnitt in demselben axialen Bereich angeordnet, insbesondere in dem zweiten axialen Bereich. Typischerweise weisen der erste Längsabschnitt und der zweite Längsabschnitt eines Stegs jeweils ein erstes Ende und ein zweites Ende auf. Bei Ausführungsformen sind die ersten Enden jeweils mit einem der benachbarten Sensorabschnitte verbunden. Beispielsweise kann ein erstes Ende eines ersten Längsabschnitts mit einem ersten Sensorabschnitt der benachbarten Sensorabschnitte verbunden sein, und ein erstes Ende des zweiten Längsabschnitts mit einem zweiten Sensorabschnitt der benachbarten Sensorabschnitte. Typischerweise sind die zweiten Enden des ersten Längsabschnitts und des zweiten Längsabschnitts über einen Umlenkabschnitt des Stegs miteinander verbunden. Der Umlenkabschnitt kann eine Umlenkung um mindestens 90° bereitstellen, insbesondere um mindestens 120° oder um mindestens 150°. Bei typischen Ausführungsformen stellt der Umlenkabschnitt eine Umlenkung von zumindest im Wesentlichen 180° bereit. Insbesondere kann der Steg im Wesentlichen U-förmig ausgebildet sein, wobei der erste Längsabschnitt und der zweite Längsabschnitt die Schenkel der U-Form bilden. Bei Ausführungsformen kann der Umlenkabschnitt beispielsweise als ein bogenförmiger Abschnitt des Stegs ausgebildet sein, insbesondere als bogenförmiger Abschnitt zwischen den zweiten Enden der ersten und zweiten Längsabschnitte. Insbesondere kann der Umlenkabschnitt als 180° Bogen ausgebildet sein, beispielsweise in einem U-förmigen Steg. Bei weiteren Ausführungsformen kann der Steg eine andere Form aufweisen, beispielsweise eine V-Form mit den ersten und zweiten Längsabschnitten als Schenkel, wobei der Umlenkabschnitt eine Umlenkung kleiner als 180° bereitstellt.

Gemäß typischen Ausführungsformen verbinden die Stege gelenkartig jeweils zwei benachbarte Sensorabschnitte. Typischerweise sind die Stege flexibel ausgebildet. Typischerweise sind die Stege jeweils als Festkörpergelenk mit einer zu der ersten Achse parallelen Gelenkachse ausgebildet.

Bei typischen Ausführungsformen sind der erste Längsabschnitt und der zweite Längsabschnitt tordiert. Insbesondere ändert sich eine Oberflächenausrichtung eines tordierten Längsabschnitts von dem ersten Ende des Längsabschnitts zu dem zweiten Ende des Längsabschnitts. Der erste Längsabschnitt und der zweite Längsabschnitt können insbesondere entlang einer axialen Richtung gegengleich tordiert sein. Der erste Längsabschnitt und der zweite Längsabschnitt können hierin auch als erste und zweite Torsionszonen des Stegs bezeichnet werden. Bei Ausführungsformen kann vorteilhafterweise eine Flexibilität des Stegs über eine Länge der Längsabschnitte eingestellt werden. Weiter können hierin beschrieben Stege ein "Federn" bzw. eine Flexibilität in radialer Richtung bereitstellen, was insbesondere eine Montage der Sensoranordnung erleichtern kann.

Typischerweise sind die Sensorabschnitte wesentlich biegesteifer als die Stege. Bei Anordnung der Sensorabschnitte um die erste Achse, beispielsweise durch Aufrollen der Sensorabschnitte und der Stege nach hierin beschriebenen Ausführungsformen, werden benachbarte Sensorabschnitte typischerweise in einem Winkel zueinander angeordnet. Die Stege stellen typischerweise die Verbindung über den Winkel zwischen den benachbarten Sensorabschnitte bereit. Im Gegensatz zu einem sich lediglich in Umfangsrichtung erstreckenden Steg, welcher um den Winkel gebogen würde, können die Stege gemäß hierin beschriebenen Ausführungsformen eine höhere Flexibilität bereitstellen. Insbesondere kann die Verbindung über den Winkel zwischen den benachbarten Sensorabschnitten im Wesentlichen durch eine Torsion der ersten und zweiten Längsabschnitte bereitgestellt werden. Beispielsweise können die erste Torsionszone und die zweite Torsionszone jeweils eine Winkeländerung von ungefähr der Hälfte des Winkels zwischen den benachbarten Sensorabschnitten bereitstellen. Die Verbindungen eines Stegs zu den Sensorabschnitten oder der Umlenkabschnitt erfahren typischerweise nur eine geringe Biegebelastung um die Gelenkachse eines als Festkörpergelenk ausgebildeten Stegs. Typischerweise ist der Umlenkabschnitt eines Stegs im Wesentlichen nicht tordiert.

Hierin beschriebene Ausführungsformen können den Vorteil haben, dass eine erhöhte Flexibilität der Verbindung zwischen benachbarten Sensorabschnitten bereitgestellt wird. Insbesondere können die Sensorabschnitte in kleinerem Krümmungsradius um die erste Achse angeordnet werden. Beispielsweise kann ein Außendurchmesser der Sensoranordnung, gemessen senkrecht zu der ersten Achse, verringert werden. Zusätzlich oder alternativ dazu kann eine erhöhte Flexibilität eine größere Beweglichkeit der Sensorabschnitte während der Montage der Sensoranordnung um die erste Achse oder eine geringere mechanische Rückwirkung auf die Sensoren bereitstellen.

Bei typischen Ausführungsformen umfasst die Sensoranordnung eine elektrische Zuleitung. Die elektrische Zuleitung umfasst typischerweise Leiterbahnen zum Betrieb der Sensoren der Sensoranordnung, insbesondere zur Energieversorgung oder zum Datenaustausch mit den Sensoren. Die elektrische Zuleitung erstreckt sich typischerweise teilweise außerhalb eines axialen Bereichs der Sensorabschnitte. Bei Ausführungsformen ist die elektrische Zuleitung in Umfangsrichtung zwischen zwei benachbarten Sensorabschnitten angeordnet. Gemäß typischen Ausführungsformen ist die elektrische Zuleitung an einem der Stege angeordnet, typischerweise an genau einem der Stege. Insbesondere kann die elektrische Zuleitung elektrisch direkt mit Leiterbahnen verbunden sein, welche in dem Steg verlaufen, an welchem die elektrische Zuleitung angeordnet ist. Beispielsweise verlaufen in Ausführungsformen, in welchen der Steg und die elektrische Zuleitung als Bereiche einer flexiblen Leiterplatte ausgebildet sind, Leiterbahnen der flexiblen Leiterplatte durchgehend von der elektrischen Zuleitung in den Steg und insbesondere weiter zu den Sensorabschnittsbereichen. Typischerweise verläuft die elektrische Zuleitung im Wesentlichen in axialer Richtung, insbesondere in einem axialen Bereich der Sensorabschnitte. Bei typischen Ausführungsformen ist die elektrische Zuleitung als Teil einer flexiblen Leiterplatte der Sensoranordnung ausgebildet, insbesondere als Teil einer flexiblen Leiterplatte, welche Stege und Sensorabschnittsbereiche ausbildet.

Bei Ausführungsformen ist die elektrische Zuleitung an einem Umlenkabschnitt eines der Stege angeordnet, beispielsweise an einem bogenförmigen Abschnitt des Steges. Insbesondere können die elektrische Zuleitung und der Umlenkabschnitt im Wesentlichen Y-förmig angeordnet sein, wobei die elektrische Zuleitung dem unteren Zweig des Y entspricht und die zwei oberen Zweige des Y dem Umlenkabschnitt entsprechen, beispielsweise einem 180° bogenförmigen Umlenkabschnitt. Bei Ausführungsformen kann eine Anordnung an einem Umlenkabschnitt den Vorteil haben, dass eine Flexibilität des Stegs, an welchem die elektrische Zuleitung angeordnet ist, nicht durch die elektrische Zuleitung beeinträchtigt wird. Insbesondere wird bei typischen Ausführungsformen der Umlenkabschnitt nicht gebogen oder tordiert, so dass eine Flexibilität, welche sich durch eine Torsion der ersten und zweiten Längsabschnitte ergibt, unbeeinflusst bleibt.

Bei typischen Sensoranordnungen verbinden die Stege die Sensorabschnitte mechanisch in Reihe gemäß hierin beschriebenen Ausführungsformen. Typischerweise ist die elektrische Zuleitung an einem in der Reihe zentral angeordneten Steg vorgesehen, insbesondere zentral in der Reihe zwischen einem Anfangssensorabschnitt und einem Endsensorabschnitt, welche nicht direkt durch einen Steg verbunden sind. Als zentral angeordneter Steg ist bei einer geraden Anzahl von Sensorabschnitten der in der Reihe zentrale Steg zu verstehen. Bei ungerader Anzahl von Sensorabschnitten kann die elektrische Zuleitung an einem der zwei in der Reihe zentral angeordneten Stege vorgesehen sein. Durch eine zentrale Anordnung der elektrischen Zuleitung kann insbesondere die Anzahl von Leiterbahnen in den einzelnen Stegen reduziert werden. Beispielsweise können die Leiterbahnen zu den auf der Seite des Anfangssensorabschnitts gelegenen Sensorabschnitten über den ersten Längsabschnitt des zentral angeordneten Stegs geführt werden, und die Leiterbahnen zu den auf der Seite des Endsensorabschnitts gelegenen Sensorabschnitten über den zweiten Längsabschnitt des zentral angeordneten Stegs. Beispielsweise kann die Anzahl der Leiterbahnen durch einzelne Stege halbiert werden. Durch eine Reduktion der Anzahl der Leiterbahnen in einem Steg wird der Steg flexibler. Weiterhin kann ein Steg bei reduzierter Anzahl von Leiterbahnen schmaler ausgeführt werden, wodurch insbesondere wiederum die Flexibilität des Stegs erhöht werden kann.

Bei weiteren Ausführungsformen kann die elektrische Zuleitung an einem anderen der Stege angeordnet sein. Bei noch weiteren Ausführungsformen kann die elektrische Zuleitung an dem Anfangssensorabschnitt oder dem Endsensorabschnitt angeordnet sein. Insbesondere kann eine elektrische Zuleitung einen Zuleitungssteg zur Anbindung der elektrischen Zuleitung an den Anfangssensorabschnitt oder den Endsensorabschnitt aufweisen. Der Zuleitungssteg kann einen Umlenkabschnitt und einen oder zwei Längsabschnitte wie hierin beschriebene Stege aufweisen. Beispielsweise kann der Zuleitungssteg im Wesentlichen U-förmig ausgebildet sein. Der Zuleitungssteg kann in einem Zwischenraum zwischen dem Anfangssensorabschnitt oder dem Endsensorabschnitt angeordnet sein. Der Zuleitungssteg kann eine höhere Flexibilität der elektrischen Zuleitung bereitstellen, beispielsweise um eine mechanische Rückwirkung auf den Anfangssensorabschnitt oder den Endsensorabschnitt zu reduzieren.

Bei typischen Ausführungsformen umfasst die Sensoranordnung zwei Deckel, insbesondere einen ersten Deckel und einen zweiten Deckel, wobei die Sensorabschnitte jeweils zumindest teilweise axial zwischen dem ersten Deckel und dem zweiten Deckel angeordnet sind. Die Deckel können beispielsweise als Scheiben ausgebildet sein, insbesondere als koaxial mit der ersten Achse angeordnete Scheiben. Die Scheiben können beispielsweise im Wesentlichen kreisrund ausgebildet sein. Bei typischen Ausführungsformen können die Deckel oder die Messkörper der Sensorabschnitte beispielsweise aus Metall hergestellt sein.

Typischerweise weist einer der Deckel eine Zuleitungsausnehmung auf, insbesondere zur Führung der elektrischen Zuleitung in axialer Richtung. Typischerweise ist die Zuleitungsausnehmung in einer radial äußeren Fläche des Deckels vorgesehen, beispielsweise als Schlitz oder Nut. Bei weiteren Ausführungsformen kann die Zuleitungsausnehmung als axiale Öffnung oder Durchgangsöffnung im Deckel ausgebildet sein. Bei typischen Ausführungsformen ist die Zuleitungsausnehmung in dem Deckel zwischen zwei in Umfangsrichtung benachbarten Sensorabschnitten vorgesehen. Die elektrische Zuleitung ist typischerweise in der Zuleitungsausnehmung angeordnet, insbesondere in axialer Richtung durch die Zuleitungsausnehmung verlaufend angeordnet. Die Anordnung der elektrischen Zuleitung und der Zuleitungsausnehmung in Umfangsrichtung zwischen benachbarten Sensorabschnitten kann den Vorteil haben, dass der Deckel nicht in einem Bereich der Sensorabschnitte durch die Zuleitungsausnehmung geschwächt wird, insbesondere bei Ausführungsformen, in denen der Deckel weitere Öffnungen oder Ausnehmungen zur Aufnahme von Zapfen der Sensorabschnitte aufweist. Insbesondere kann eine Stabilität der Sensoranordnung erhöht werden. Alternativ kann im Vergleich zu einer Ausführung ohne eine Zuleitungsausnehmung ein Biegen der elektrischen Zuleitung vermieden werden. Weiter können Ausführungsformen den Vorteil haben, dass die elektrische Zuleitung und die Zuleitungsausnehmung abseits einer Schweißnaht zwischen Zapfen und Deckel angeordnet sind, was insbesondere die Montage der Sensoranordnung erleichtert.

Bei beispielhaften Ausführungsformen können 6 Sensorabschnitte hexagonal um die erste Achse zwischen zwei Deckeln angeordnet werden, insbesondere zur Ausbildung eines Hexapods. Beispielsweise können unter Verwendung der Sensoranordnung drei verschiedene Kraftkomponenten oder drei verschiedene Drehmomentkomponenten unabhängig voneinander messbar sein, insbesondere drei verschiedene Kraftkomponenten und drei verschiedene Drehmomentkomponenten.

Gemäß typischen Ausführungsformen umfassen die Sensorabschnitte jeweils einen Messkörper. Der Messkörper kann beispielsweise im Wesentlichen quaderförmig ausgebildet sein. Insbesondere kann die Struktur von aufgerollten Sensorabschnitten im Wesentlichen einem Polygon entsprechen. Typischerweise weist der Messkörper die größte Erstreckung in axialer Richtung auf. Typischerweise umfasst der Messkörper einen Schwächungsbereich. Typischerweise ist der Messkörper in dem Schwächungsbereich verjüngt ausgebildet, insbesondere in einem Bereich des Sensors des Sensorabschnitts. Der Schwächungsbereich kann mindestens eine Schwächungsausnehmung aufweisen, insbesondere zwei Schwächungsausnehmungen. Eine Schwächungsausnehmung kann beispielsweise als Öffnung, Bohrung oder Ausfräsung in dem Messkörper ausgebildet sein, z.B. als kreisrunde Bohrung oder als L-förmige oder C-förmige Ausfräsung. Typischerweise umfasst der Messkörper eine der ersten Achse zugewandte erste Seite. Bei typischen Ausführungsformen ist die mindestens eine Schwächungsausnehmung zumindest im Wesentlichen senkrecht durch die erste Seite vorgesehen.

Typischerweise weist der Messkörper in dem Schwächungsbereich eine Brücke auf, welche Teile des Messkörpers zwischen einem ersten axialen Ende und einem zweiten axialen Ende des Schwächungsbereichs miteinander verbindet. Die Brücke kann insbesondere zwischen zwei Schwächungsausnehmungen verlaufen. Die Schwächungsausnehmungen können derart angeordnet sein, dass die zwischen den Schwächungsausnehmungen verlaufende Brücke einen Winkel mit einer axialen Richtung einschließt, beispielsweise einen Winkel von mindestens 30°, insbesondere von mindestens 35° oder von mindestens 40°, oder von maximal 60°, insbesondere von maximal 55°.

Bei Ausführungsformen können die Messkörper oder die Brücken der Sensorabschnitte zueinander geneigt angeordnet werden. Beispielsweise können in einer Sensoranordnung mit 6 Sensorabschnitten die Brücken des Messkörpers hexagonal um die erste Achse angeordnet werden. Die Brücken können geneigt, insbesondere relativ zueinander geneigt, zwischen zwei Deckeln angeordnet werden, insbesondere zur Ausbildung eines Hexapods.

Typischerweise ist der Sensor eines Sensorabschnitts in dem Schwächungsbereich des Sensorabschnitts angeordnet, insbesondere auf der Brücke des Schwächungsbereichs. In dem Schwächungsbereich können insbesondere Dehnungen des Messkörpers präzise durch den Sensor gemessen werden. Bei Ausführungsformen umfasst der Messkörper typischerweise einen im Wesentlichen starren Aufnahmebereich zur Aufnahme des Sensorabschnittsbereichs der flexiblen Leiterplatte. Der Aufnahmebereich kann axial versetzt zu dem Schwächungsbereich vorgesehen sein. Typischerweise ist die Verdrahtungszone eines Sensorabschnittsbereichs einer flexiblen Leiterplatte auf dem Aufnahmebereich des Messkörpers angeordnet. Typischerweise erstrecken sich die Stege zumindest im Wesentlichen innerhalb des gleichen axialen Bereichs wie der Aufnahmebereich des Messkörpers.

Bei typischen Ausführungsformen umfasst ein Sensorabschnitt eine Sensorzuleitung, insbesondere eine Sensorzuleitung zur elektrischen Verbindung zwischen dem Sensor und einem Sensorabschnittsbereich einer flexiblen Leiterplatte. Bei Ausführungsformen kann die Sensorzuleitung durch Bonddrähte zwischen dem Sensor und dem Sensorabschnittsbereich bereitgestellt werden. Die Bonddrähte können beispielsweise elektrisch mit Kontaktpads des Sensorabschnittsbereichs verbunden werden. Bei weiteren Ausführungsformen kann die Sensorzuleitung als Sensorzuleitungsbereich der flexiblen Leiterplatte ausgebildet sein. Ein Sensorzuleitungsbereich kann beispielsweise über einen oder mehrere Lötpunkte mit einem Sensor verbunden werden. Der Sensorzuleitungsbereich kann als Sensorzuleitungssteg mit mehreren Bögen oder mäanderförmig ausgebildet sein, insbesondere um eine hohe Flexibilität oder geringe mechanische Wirkung zwischen dem Sensorabschnittsbereich der flexiblen Leiterplatte und dem Sensor bereitzustellen.

Bei typischen Ausführungsformen umfassen die Sensorabschnitte, insbesondere die Messkörper der Sensorabschnitte, jeweils mindestens einen Zapfen an einem axialen Ende des jeweiligen Sensorabschnitts. Typischerweise umfassen die Sensorabschnitte jeweils zwei Zapfen, insbesondere je einen Zapfen an je einem der zwei axialen Enden eines Sensorabschnitts. Typischerweise sind die Zapfen eingerichtet zum Eingriff in den ersten Deckel oder den zweiten Deckel der Sensoranordnung. Der erste Deckel oder der zweite Deckel können entsprechende Öffnungen oder Ausnehmungen zur Aufnahme der Zapfen der Sensorabschnitte aufweisen. Eine feste Verbindung zwischen den Zapfen und Deckel kann beispielsweise durch eine Steckverbindung zwischen Zapfen und Deckel oder durch eine Schweißnaht zwischen Zapfen und Deckel bereitgestellt werden, insbesondere durch eine Steckverbindung und eine Schweißnaht.

Bei typischen Ausführungsformen weist die Sensoranordnung einen Durchmesser senkrecht zu der ersten Achse von höchstens 15 mm auf, insbesondere von höchstens 10 mm oder von höchstens 8 mm. Beispielsweise kann die Sensoranordnung einen Durchmesser von ungefähr 8 mm oder von ungefähr 6 mm aufweisen. Der Durchmesser bezieht sich auf einen Außendurchmesser. In anderen Ausführungsformen weist die Sensoranordnung einen Durchmesser senkrecht zu der ersten Achse von höchstens 21°mm oder 32°mm auf. Hierin beschriebene Ausführungsformen stellen beispielsweise eine erhöhte Flexibilität der Stege oder eine erhöhte Stabilität der Verbindung von Deckel und Sensorabschnitten bereit, insbesondere zur Miniaturisierung von hierin beschriebenen Sensoranordnungen zur Kraft- oder Drehmomentmessung. Bei typischen Ausführungsformen ist eine axiale Länge der Sensoranordnung kleiner als 20 mm, insbesondere kleiner als 15 mm, insbesondere ohne Berücksichtigung einer axialen Erstreckung einer elektrischen Zuleitung.

Gemäß typischen Ausführungsformen ist ein Verfahren zum Herstellen einer Sensoranordnung angegeben, insbesondere einer Sensoranordnung gemäß hierin beschriebenen Ausführungsformen. Das Verfahren umfasst ein Bereitstellen der Mehrzahl von Sensorabschnitten, welche über die Stege verbunden sind. Typischerweise sind die Sensorabschnitte in einer Ebene angeordnet, insbesondere in einer Ebene parallel zu einer ersten Achse. Typischerweise umfasst das Bereitstellen der Sensorabschnitte und Stege ein Bereitstellen von Messkörpern gemäß hierin beschriebenen Ausführungsformen, insbesondere mit jeweils einem Sensor, welcher auf oder in dem Messkörper angeordnet ist. Eine flexible Leiterplatte kann mit Sensorabschnittsbereichen und zwischen den Sensorabschnittsbereichen angeordneten Stegen gemäß hierin beschriebenen Ausführungsformen bereitgestellt werden. Die flexible Leiterplatte kann in der Ebene angeordnet werden. Die Messkörper können fest mit jeweiligen Sensorabschnittsbereichen der flexiblen Leiterplatte verbunden werden. Die Sensoren können elektrisch mit den jeweiligen Sensorabschnittsbereichen verbunden werden. Durch die parallele Anordnung der Sensorabschnitte in einer Ebene können diese Herstellungsschritte beispielsweise kostengünstig vorgenommen werden.

Typischerweise umfasst das Verfahren ein Aufrollen der Sensorabschnitte zur Anordnung der Sensorabschnitte um die erste Achse. Bei dem Aufrollen der Sensorabschnitte können die Sensorabschnitte in einem Winkel zueinander angeordnet werden, wobei insbesondere die Stege als Festkörpergelenke zwischen benachbarten Sensorabschnitten dienen. Typischerweise sind die Stege vor dem Aufrollen nicht tordiert. Typischerweise werden die Stege, insbesondere jeweils ein erster Längsabschnitt und ein zweiter Längsabschnitt der Stege, während des Aufrollens tordiert.

Bei typischen Ausführungsformen umfasst das Verfahren ein Verbinden der Sensorabschnitte mit mindestens einem Deckel, insbesondere mit einem ersten Deckel und einem zweiten Deckel. Insbesondere können die Sensorabschnitte und der mindestens eine Deckel fest miteinander verbunden werden. Der mindestens eine Deckel kann gemäß hierin beschriebenen Ausführungsformen bereitgestellt werden. Bei Ausführungsformen werden die Sensorabschnitte, insbesondere die aufgerollten Sensorabschnitte, axial zwischen dem ersten Deckel und dem zweiten Deckel angeordnet. Die Sensorabschnitte können an den axialen Enden der Sensorabschnitte jeweils einen Zapfen aufweisen. Die Zapfen können mit Öffnungen oder Ausnehmungen des ersten Deckels und des zweiten Deckels in Eingriff gebracht werden. Zusätzlich oder alternativ dazu können die Sensorabschnitte und der mindestens eine Deckel stoffschlüssig verbunden werden, insbesondere miteinander verklebt oder verschweißt werden. Eine elektrische Zuleitung kann in einer Zuleitungsausnehmung des ersten Deckels oder des zweiten Deckels angeordnet werden.

Typische Sensoranordnungen können gegenüber dem Stand der Technik den Vorteil bieten, dass Sensoranordnungen mit geringerem Außendurchmesser hergestellt werden können. Insbesondere kann eine erhöhte Flexibilität der Stege bereitgestellt werden, wodurch beispielsweise kleine Krümmungsradien in Umfangsrichtung zwischen den Sensorabschnitten verwendet werden können. Typische Sensoranordnungen können eine geringe mechanische Rückwirkung auf die DMS-Sensorik aufweisen, insbesondere durch eine Verdrehung der Torsionszonen der Stege. Intrinsische Spannungen in der flexiblen Leiterplatte, die sich negativ auf eine Messgenauigkeit der Sensoranordnung auswirken, können reduziert werden. Insbesondere können zusätzliche, störende Kräfte vermieden werden, welche bei geringerer Flexibilität der Stege in die mit Sensoren bestückten Sensorabschnitte eingekoppelt würden. Ausführungsformen können ein verbessertes Driftverhalten aufweisen, insbesondere ein nicht lineares oder nicht reproduzierbares Temperaturverhalten vermeiden oder reduzieren. Weiter können Ausführungsformen den Vorteil bieten, dass eine große Beweglichkeit der Sensorabschnitte während der Montage bereitgestellt werden kann. Typische Ausführungsformen können weiter eine verbesserte Signalausleitung aufweisen. Insbesondere kann die elektrische Zuleitung gemäß Ausführungsformen unschädlich sein für eine Stabilität der Verbindung zwischen Sensorabschnitten und Deckel. Weiter kann sich die elektrische Zuleitung vorteilhaft in Bezug auf eine Flexibilität der Stege auswirken.

### Kurze Beschreibung der Zeichnungen

Weitere Vorteile und Merkmale bevorzugter Ausführungsformen der Erfindung werden nachfolgend anhand der beiliegenden Zeichnungen erläutert, wobei die Figuren zeigen:
- Fig. 1: zeigt eine Ansicht einer Sensoranordnung gemäß einer typischen Ausführungsform;
- Fig. 2: zeigt eine schematische Ansicht einer Mehrzahl von Sensorabschnitten, welche über Stege miteinander verbunden sind, gemäß der Ausführungsform der Fig. 1;
- Fig. 3: zeigt einen Ausschnitt der flexiblen Leiterplatte gemäß der Sensoranordnung der Fig. 1, in einer perspektivischen Ansicht von dem ersten Deckel aus axial in Richtung des zweiten Deckels;
- Figuren 4 bis 6: zeigen jeweils eine schematische Ansicht einer Mehrzahl von Sensorabschnitten und Stegen gemäß weiteren typischen Ausführungsformen;
- Fig. 7: zeigt ein Flussdiagramm eines Verfahrens zum Herstellen einer Sensoranordnung gemäß einer typischen Ausführungsform.

### Beschreibung von Ausführungsformen

Nachfolgend werden typische Ausführungsformen anhand der Figuren beschrieben, wobei die Erfindung nicht auf die Ausführungsbeispiele beschränkt ist, vielmehr wird der Umfang der Erfindung durch die Ansprüche bestimmt.

Bei der Beschreibung der Figuren werden die gleichen Bezugszeichen für gleiche oder ähnliche Teile verwendet. Teilweise werden Merkmale, welche bereits im Zusammenhang mit anderen Figuren beschrieben wurden, der Übersichtlichkeit halber nicht nochmals beschrieben.

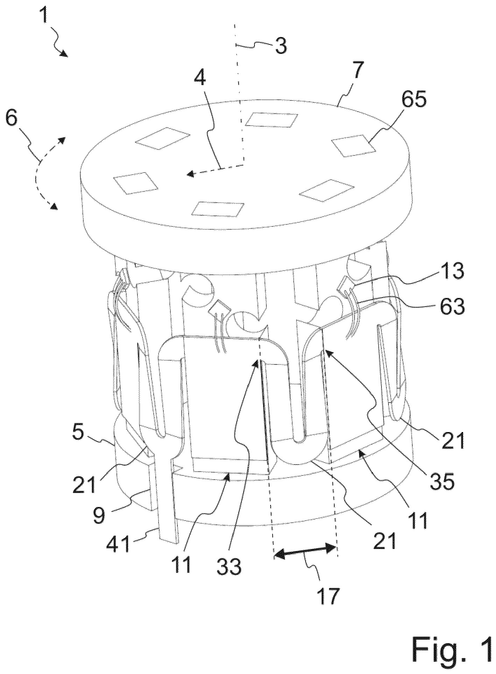

Fig. 1 zeigt eine schematische Ansicht einer Sensoranordnung 1 gemäß typischen Ausführungsformen. Die Sensoranordnung 1 ist insbesondere als Hexapod mit 6 Sensorabschnitten 11 ausgebildet. Die Sensorabschnitte 11 sind mechanisch in Reihe über Stege 21 miteinander verbunden. Die Sensorabschnitte 11 sind rings um eine erste Achse 3 angeordnet, insbesondere aufgerollt. In der Reihe benachbarte Sensorabschnitte 11 sind in Fig. 1 in einem Winkel von 60° zueinander angeordnet. Die in den Zwischenräumen 17 zwischen den Sensorabschnitten 11 angeordneten Stege 21 dienen als Festkörpergelenke zwischen den Sensorabschnitten 11. Die Sensorabschnitte 11 sind axial teilweise zwischen einem ersten Deckel 5 und einem zweiten Deckel 7 angeordnet und fest mit dem ersten Deckel 5 und dem zweiten Deckel 7 verbunden. Insbesondere greifen Zapfen 65, die jeweils an den axialen Enden der Sensorabschnitte 11 angeordnet sind, in entsprechende Öffnungen des ersten Deckels 5 und des zweiten Deckels 7 ein. Die Dicke der Stege 21 in Richtung senkrecht zur Achse 3, insbesondere in radialer Richtung 4, beträgt weniger als ein Drittel der Dicke der Sensorabschnitte 11.

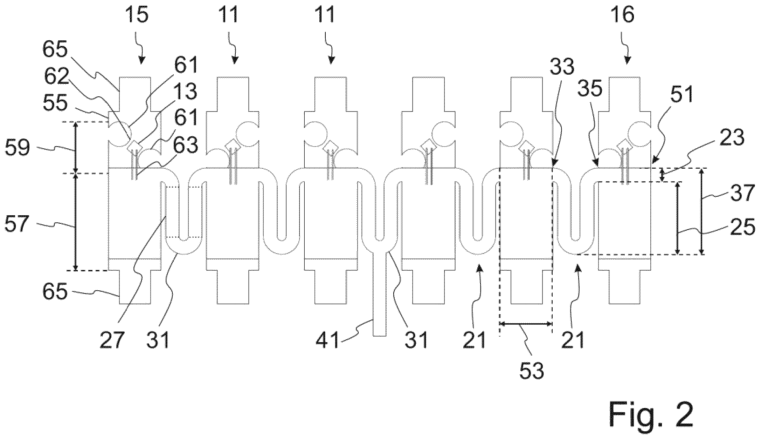

Fig. 2 stellt die Sensorabschnitte 11 und Stege 21 gemäß Fig. 1 in einem abgerollten Zustand dar, insbesondere ohne den ersten Deckel 5 und den zweiten Deckel 7. Die Sensorabschnitte 11 umfassen jeweils einen Messkörper 55, einen Sensor 13 und einen Sensorabschnittsbereich 53 einer flexiblen Leiterplatte 51, wobei der Sensorabschnittsbereich 53 fest mit dem Messkörper 55 verbunden ist. Der Messkörper 55 weist die zwei Zapfen 65 auf. Der Messkörper 55 eines Sensorabschnitts 11 umfasst einen Schwächungsbereich 59, in welchem Schwächungsausnehmungen 61, in Fig. 1 zwei Bohrungen durch den Messkörper 55, um eine Brücke 62 des Messkörpers 55 angeordnet sind. Auf der Brücke 62 ist der Sensor 13, in Figuren 1, 2 und 4 ein Si-DMS, fest angeordnet. Weiter umfasst der Messkörper 55 einen axial an den Schwächungsbereich 59 angrenzenden, relativ starren Aufnahmebereich 57, welcher fest mit dem Sensorabschnittsbereich 53 der flexiblen Leiterplatte 51 verbunden ist. Der Sensor 13 und der Sensorabschnittsbereich 53 sind elektrisch über eine Sensorzuleitung 63, in Fig. 1 Bonddrähte, miteinander verbunden.

Die flexible Leiterplatte 51 umfasst weiter die Stege 21 sowie eine elektrische Zuleitung 41 zum Betrieb der Sensoranordnung 1, insbesondere der Sensoren 13. Die Stege 21 sind jeweils mit zwei benachbarten Sensorabschnitten 11 verbunden. Die mechanisch in Reihe verbundenen Sensorabschnitte 11 umfassen einen Anfangssensorabschnitt 15 und einen Endsensorabschnitt 16, welche nicht direkt durch einen Steg 21 miteinander verbunden sind. Die Stege 21 weisen jeweils eine Verbindung 33 zu einem ersten Sensorabschnitt von zwei benachbarten Sensorabschnitten auf, sowie eine weitere Verbindung 35 zu einem zweiten Sensorabschnitt der benachbarten Sensorabschnitte. In den Ausführungsformen mit flexibler Leiterplatte 51 sind die Verbindung 33 und die weitere Verbindung 35 jeweils durch einen Übergang der flexiblen Leiterplatte 51 von einem Sensorabschnittsbereich 53 zu dem Steg 21 gebildet, insbesondere an einem dem Sensor 13 nähergelegenen axialen Ende des Sensorabschnittsbereichs 53. Die Verbindung 33 und insbesondere auch die weitere Verbindung 35 sind in einem ersten axialen Bereich 23 vorgesehen. Die Stege 21 erstrecken sich jeweils in dem Zwischenraum 17 zwischen zwei benachbarten Sensorabschnitten 11 über den ersten axialen Bereich 23 hinaus in einen von dem ersten axialen Bereich 23 verschiedenen zweiten axialen Bereich 25. Eine axiale Erstreckung 37 der Stege 21 ist größer als ein Drittel einer axialen Erstreckung der Sensorabschnitte 11.

Die Stege 21 umfassen jeweils einen ersten Längsabschnitt 27 und einen zweiten Längsabschnitt 29, welche sich in demselben axialen Bereich, insbesondere dem zweiten axialen Bereich 25, erstrecken. In dem abgerollten Zustand der Fig. 2 sind die ersten und zweiten Längsabschnitte flach angeordnet und axial ausgerichtet. Der erste Längsabschnitt 27 eines Stegs 21 und der zweite Längsabschnitt 29 des Stegs 21 sind über einen Umlenkabschnitt 31 des Stegs 21 miteinander verbunden, insbesondere durch einen 180° bogenförmigen Umlenkabschnitt 31. Insgesamt sind die Stege 21 im Wesentlichen U-förmig ausgebildet.

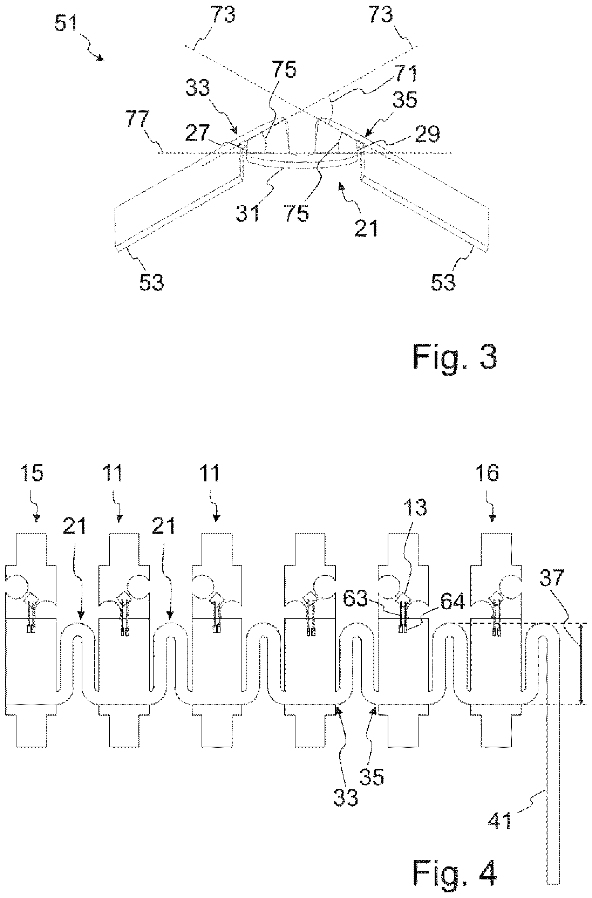

Fig. 3 zeigt eine perspektivische Ansicht eines Ausschnitts der flexiblen Leiterplatte 51 der Fig. 1, insbesondere von dem ersten Deckel 5 in Richtung des zweiten Deckels 7. Insbesondere zeigt die Fig. 3 zwei Sensorabschnittsbereiche 53 der flexiblen Leiterplatte 51, wobei die zwei Sensorabschnittsbereiche 53 in einem Winkel 71 von 60° zueinander angeordnet sind. Die zwei Sensorabschnittsbereiche 53 sind über einen Steg 21 miteinander verbunden, wobei der erste Längsabschnitt 27 und der zweite Längsabschnitt 29 des Stegs 21 tordiert sind. Der Umlenkabschnitt 31 bleibt im Wesentlich unverdreht und unverbogen. Wie in Fig. 3 schematisch dargestellt, schließt eine Linie 77 entlang der Oberfläche der Längsabschnitte am Übergang zu dem Umlenkabschnitt 31 die Winkel 75 mit Verlängerungslinien 73 der Sensorabschnittsbereiche 53 ein, wobei die Winkel 75 jeweils ungefähr 30° betragen. Insbesondere wird der Winkel 71 von 60° zwischen zwei Sensorabschnitten durch gegengleiche Torsion um 30° des ersten Längsabschnitts 27 und des zweiten Längsabschnitts 29 bereitgestellt. Der Steg 21 weist eine hohe Flexibilität, geringe Biegebelastung und eine geringe Rückwirkung auf die Sensorik auf.

Wie in Figuren 1 und 2 dargestellt, umfasst die flexible Leiterplatte 51 die elektrische Zuleitung 41, welche an einem zentral in der Reihe der Sensorabschnitte 11 angeordneten Steg 21 vorgesehen ist, insbesondere Y-förmig, an einem Umlenkabschnitt 31 des Stegs 21. Durch die zentrale Anordnung kann die Anzahl der Leiterbahnen pro Steg im Vergleich zu einer endseitigen Zuleitung verringert werden. Durch die geringere Anzahl an Leiterbahnen sind die Stege zudem flexibler. In Fig. 1 weist der erste Deckel 5 weiter eine Zuleitungsausnehmung 9 zur axialen Führung der elektrischen Zuleitung 41 zu dem Steg 21 auf. Die elektrische Zuleitung 41 und die Zuleitungsausnehmung 9 sind in Umfangsrichtung 6 zwischen zwei Sensorabschnitten angeordnet. Insbesondere ist die Zuleitungsausnehmung in Umfangsrichtung 6 versetzt zu den Ausnehmungen des ersten Deckels 5 für die Zapfen 65 der Sensorabschnitte 11 angeordnet, wodurch insbesondere eine Stabilität des ersten Deckels 5 erhöht wird.

Fig. 4 zeigt eine Mehrzahl von Sensorabschnitten 11 und Stegen 21 in abgerolltem Zustand für eine Sensoranordnung gemäß einer weiteren Ausführungsform. In Fig. 4 sind die Verbindung 33 und die weitere Verbindung 35 zwischen den Stegen 21 und den Sensorabschnitten 11 jeweils an einem von dem Sensor 13 weiter entfernt gelegenen axialen Ende des Sensorabschnittsbereichs 53 vorgesehen. Weiter ist die elektrische Zuleitung 41 endseitig angeordnet, insbesondere an dem Endsensorabschnitt 16. Die elektrische Zuleitung 41 umfasst einen U-förmigen Zuleitungssteg, welcher ähnlich wie die Stege 21 ausgebildet ist. Weiter sind in Fig. 4 auf den Sensorabschnittsbereichen 53 jeweils beispielsweise zwei Kontaktpads 64 dargestellt, welche zur Verbindung mit den Sensoren 13 über Bonddrähte (Sensorzuleitung 63) eingerichtet sind. In anderen Ausführungsformen können auch beispielsweise drei, vier oder fünf Kontaktpads vorhanden sein.

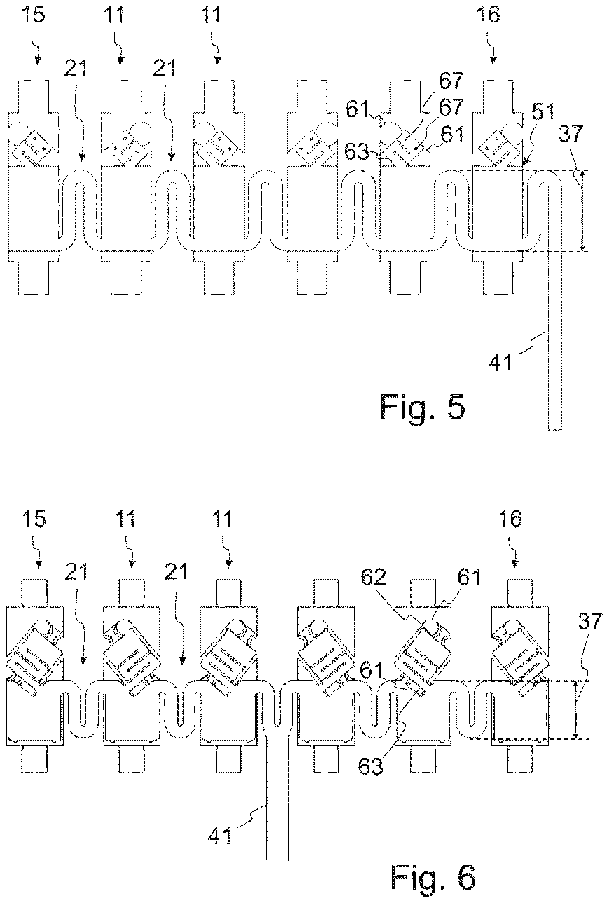

Fig. 5 zeigt eine Mehrzahl von Sensorabschnitten 11 und Stegen 21 in abgerolltem Zustand für eine Sensoranordnung gemäß noch einer weiteren Ausführungsform. In Fig. 5 ist die Sensorzuleitung 63 als Teil der flexiblen Leiterplatte 51 ausgebildet. Insbesondere umfassen die Sensorabschnitte 11 jeweils eine Sensorzuleitung 63, welche als mäanderförmiger Sensorzuleitungssteg zwischen dem Sensorabschnittsbereich 53 des Sensorabschnitts 11 zu dem Sensor (in Figuren 5 und 6 durch die Sensorzuleitung 63 verdeckt) ausgebildet ist. Die Sensoren der Figuren 5 und 6 sind beispielsweise Folien-DMS. Eine elektrische Verbindung zwischen der Sensorzuleitung 63 und dem Sensor ist über Lötpunkte 67 bereitgestellt. Ähnlich wie in Fig. 4 ist die elektrische Zuleitung 41 endseitig angeordnet.

Fig. 6 zeigt eine Mehrzahl von Sensorabschnitten 11 und Stegen 21 in abgerolltem Zustand für eine Sensoranordnung gemäß einer weiteren Ausführungsform. In Fig. 6 weisen die Sensorabschnitte 11 jeweils zwei Schwächungsausnehmungen 61 auf, welche im Wesentlichen geneigt C-förmig um eine Brücke 62 ausgefräst sind. Eine axiale Erstreckung 37 der Stege 21 ist in Fig. 5 beispielsweise ungefähr ein Drittel der axialen Erstreckung der Sensorabschnitte 11. Ähnlich wie in Fig. 2 ist die elektrische Zuleitung 41 an einem in der Reihe der Sensorabschnitte 11 zentralen Steg 21 angeordnet.

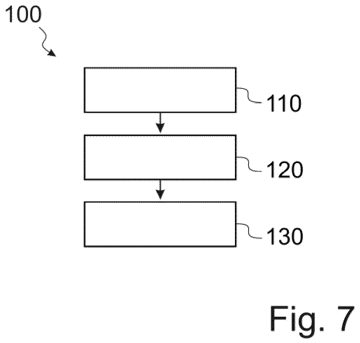

Fig. 7 zeigt ein Flussdiagramm eines Verfahrens 100 zum Herstellen einer Sensoranordnung 1 gemäß hierin beschriebenen Ausführungsformen. Bei Block 110 umfasst das Verfahren 100 ein Bereitstellen der Mehrzahl von Sensorabschnitten 11, welche über die Stege 21 verbunden sind, wobei die Sensorabschnitte 11 in einer Ebene angeordnet sind. Bei Block 120 umfasst das Verfahren 100 ein Aufrollen der Sensorabschnitte 11 zur Anordnung der Sensorabschnitte 11 um eine erste Achse 3. Bei Block 130 umfasst das Verfahren 100 ein Verbinden der Sensorabschnitte 11 mit einem ersten Deckel 5 und einem zweiten Deckel 7. Durch die hohe Flexibilität der Stege kann die Herstellung der Sensoranordnung und insbesondere das Aufrollen der Sensorabschnitte verbessert werden. Beispielsweise kann bei Ausführungsformen ein Ablösen der flexiblen Leiterplatte von Messkörpern der Sensorabschnitte vermieden oder eine Rückwirkung der Stege auf die Sensoren der Sensorabschnitte verringert werden.

## Patentansprüche

1. Sensoranordnung (1) zur Messung einer Kraft und/oder eines Drehmoments, umfassend
eine Mehrzahl von Sensorabschnitten (11) mit jeweils einem Sensor (13), wobei die Sensorabschnitte (11) um eine erste Achse (3) angeordnet ist; und
eine Mehrzahl von Stegen (21), welche jeweils zwei benachbarte der Sensorabschnitte (11) miteinander verbinden, wobei eine Verbindung (33) zwischen einem Steg (21) und einem ersten Sensorabschnitt von benachbarten Sensorabschnitten in einem ersten axialen Bereich (23) bereitgestellt ist, und wobei sich der Steg (21) in einem Zwischenraum (17) zwischen den benachbarten Sensorabschnitten in einen von dem ersten axialen Bereich (23) verschiedenen zweiten axialen Bereich (25) erstreckt.

2. Sensoranordnung (1) nach Anspruch 1, wobei die Stege (21) jeweils einen sich axial erstreckenden ersten Längsabschnitt (27) und einen sich axial erstreckenden zweiten Längsabschnitt (29) aufweisen, und wobei der erste Längsabschnitt (27) und der zweite Längsabschnitt (29) in demselben axialen Bereich angeordnet sind.

3. Sensoranordnung (1) nach Anspruch 2, wobei der erste Längsabschnitt (27) und der zweite Längsabschnitt (29) tordiert sind.

4. Sensoranordnung (1) nach Anspruch 2 oder 3, wobei der erste Längsabschnitt (27) und der zweite Längsabschnitt (29) eines Stegs (21) jeweils ein erstes Ende und ein zweites Ende aufweisen, wobei die ersten Enden jeweils mit einem der benachbarten Sensorabschnitte (11) verbunden sind, und wobei die zweiten Enden über einen Umlenkabschnitt (31) des Stegs (21) miteinander verbunden sind.

5. Sensoranordnung (1) nach einem der vorhergehenden Ansprüche, wobei eine weitere Verbindung (35) zwischen dem Steg (21) und einem zweiten Sensorabschnitt der benachbarten Sensorabschnitte in dem ersten axialen Bereich (23) bereitgestellt ist.

6. Sensoranordnung (1) nach einem der vorhergehenden Ansprüche, wobei die Stege (21) jeweils als Festkörpergelenk mit einer zu der ersten Achse (3) parallelen Gelenkachse ausgebildet sind.

7. Sensoranordnung (1) nach einem der vorhergehenden Ansprüche, wobei sich die Stege (21) jeweils in axialer Richtung über mindestens ein Viertel einer axialen Länge eines Sensorabschnittes (11) erstrecken.

8. Sensoranordnung (1) nach einem der vorhergehenden Ansprüche, wobei die Sensoranordnung (1) weiter eine elektrische Zuleitung (41) umfasst, wobei die elektrische Zuleitung (41) an einem der Stege (21) angeordnet ist.

9. Sensoranordnung (1) nach Anspruch 8, wobei die elektrische Zuleitung (41) an einem Umlenkabschnitt (31) eines der Stege (21) angeordnet ist.

10. Sensoranordnung (1) nach Anspruch 8 oder 9, wobei die Stege die Sensorabschnitte mechanisch in Reihe verbinden, und wobei die elektrische Zuleitung (41) an einem in der Reihe zentral angeordneten Steg vorgesehen ist.

11. Sensoranordnung (1) nach einem der vorhergehenden Ansprüche, umfassend eine flexible Leiterplatte (51) mit Sensorabschnittsbereichen (53) und Stegbereichen, wobei die Sensorabschnitte (11) jeweils einen der Sensorabschnittsbereiche (53) der flexiblen Leiterplatte (51) umfassen, und wobei die Stegbereiche der flexiblen Leiterplatte (51) die Stege (21) bereitstellen.

12. Sensoranordnung (1) nach einem der vorhergehenden Ansprüche, weiter umfassend einen ersten Deckel (5) und einen zweiten Deckel (7), wobei die Sensorabschnitte (11) jeweils zumindest teilweise axial zwischen dem ersten Deckel (5) und dem zweiten Deckel (7) angeordnet sind.

13. Sensoranordnung (1) nach einem der vorhergehenden Ansprüche, wobei die Mehrzahl von Sensorabschnitten (11) rings um die erste Achse (3) angeordnet sind.

14. Sensoranordnung (1) nach einem der vorhergehenden Ansprüche, wobei die Sensoranordnung (1) einen Durchmesser senkrecht zu der ersten Achse (3) von höchstens 15 mm aufweist.

15. Verfahren (100) zum Herstellen einer Sensoranordnung (1) gemäß einem der vorhergehenden Ansprüche, mit
Bereitstellen der Mehrzahl von Sensorabschnitten (11), welche über die Stege (21) verbunden sind, wobei die Sensorabschnitte (11) in einer Ebene angeordnet sind; und
Aufrollen der Sensorabschnitte (11) zur Anordnung der Sensorabschnitte (11) um die erste Achse (3).
